# EUROPEAN PATENT APPLICATION

(11) **EP 3 292 836 A1**
(43) Date of publication of application: **14.03.2018**
(21) Application number: 16849918.4
(22) Date of filing: 20.04.2016
(51) Int. Cl.: A61B 42/30, A61B 42/50

(54) **ELASTIC GLOVE TESTING AND DONNING DEVICE WITH STERILIZATION FUNCTION**

(30) Priority: 14.02.2016 CN 201620119646 U; 15.04.2016 CN 201610235010
(71) Applicant: Xi'An Libang Medical Electr. Co., Ltd., Xi'An, Shaanxi 710077 (CN)
(72) Inventor: JU, Junwu, Xi'an Shaanxi 710077 (CN)
(74) Representative: Gulde & Partner
(86) International application number: PCT/CN2016/079765
(87) International publication number: WO 2017/136974

(57) **Abstract**

The invention relates to equipment for elastic glove airtightness-detecting and rapid-donning with a sterilization function. The equipment for elastic glove airtightness-detecting and rapid-donning with the sterilization function comprises two sterilization cavities internally provided with sterilization devices, wherein through holes used for mounting sleeve fixing rings for elastic gloves are formed in one side of the sterilization cavities; the fingerstall section of the gloves are located inside the sterilization cavities, the sleeve ends of the gloves are fixed to the sterilization cavities through the sleeve fixing rings, and the gloves can be donned rapidly under control action; the two sides of the bottom of each sterilization cavity are provided with air pipeline ports connected with a two-way air pump serving as an air source, and small-power ozone generation pieces which serve as the sterilization devices and pressure detection switches which are used for detecting the internal pressure of the sterilization cavities and conducting closed-loop control are also mounted; air pipelines are provided with electromagnetic valves, flow regulating valves, one-way valves and sterilizing filters and connected to the sterilization cavities and the air pump; the wearer treads on an external pedal switch to generate negative pressure, balanced pressure and positive pressure in the sterilization cavities, and thus the elastic gloves are made to contract and automatically fall onto the hands of the wearer from the sleeve fixing rings after being done.

## Description

### BACKGROUND OF THE INVENTION

### Technical Field

The invention relates to elastic glove donning equipment, in particular to equipment for airtightness-detecting, sterilizing and donning of elastic gloves, and belongs to the technical field of medical sterilizing equipment.

### 2. Description of Related Art

Elastic gloves are indispensable articles for medical operation. Generally, coordination of nurses or other personnel is needed when the elastic gloves are donned at present, the gloves are difficult to don, the exterior surfaces of the gloves can be contaminated and damaged easily, and consequentially medical negligence such as cross infection can be caused. Meanwhile, since non-plastic elastic gloves generally have good elasticity, the non-plastic elastic gloves are more difficult to don and cannot be donned and used in emergencies such as nursing during a physical examination or a surgical operation. Part of the existing auxiliary equipment technically provides a certain convenience for donning the elastic gloves, however, the existing auxiliary equipment needs to be controlled manually and does not have the glove airtightness automatic-detecting function, the sterilization function and the like, and consequentially medical negligence can be caused easily.

### BRIEF SUMMARY OF THE INVENTION

The invention aims to overcome the defects in the prior art and provides equipment for elastic glove airtightness-detecting and donning with a sterilization function, which is reasonable in structural design, fast and convenient to use, and capable of effectively reducing cross infection.

According to the technical scheme of the invention:
The equipment for elastic glove detecting and donning with the sterilization function comprises sterilization cavities, wherein elastic gloves are fixedly mounted in the sterilization cavities, fingerstall sections of the elastic gloves are located inside the sterilization cavities, cavity through holes used for mounting sleeve fixing rings are formed in one side of the sterilization cavities, and the sleeve ends are fixed to the sterilization cavities through the sleeve fixing rings; the sterilization cavities are further provided with pressure detection switches which are used for detecting the internal pressure of the sterilization cavities and also used for closed-loop control; the sterilization cavities are further provided with air pipeline ports used for being connected with an air pump, the air pump is connected with the sterilization cavities through electromagnetic valves, the sterilization cavities and the electromagnetic valves are further connected with a control cabinet, and the electromagnetic valves are controlled through the control cabinet, so that the elastic gloves are stretched to be donned under negative pressure and contracted to be released under positive pressure.

Sterilization devices are arranged in each sterilization cavity and include an ozone generation device, a pasteurization device, an ultraviolet sterilization device and a negative ion sterilization device.

The equipment for elastic glove airtightness-detecting and rapid-donning with the sterilization function further comprises air suction cycles used for forming negative-pressure areas in the airtight areas between the interiors of the sterilization cavities and the exteriors of the gloves to make the gloves expand to become larger, and air inflation cycles used for forming positive-pressure areas in the airtight areas between the interiors of the sterilization cavities and the exteriors of the gloves to attach the gloves completely to human hands; an air inlet of the air pump is connected with ports A of the electromagnetic valves and communicates with air outlets of the sterilization cavities through an air suction pipeline, and an air outlet of the air pump is connected with ports B of the electromagnetic valves and communicates with the outside atmosphere, so that the air suction cycles are formed; the air outlet of the air pump is connected with the ports B of the electromagnetic valves and communicates with air inlets of the sterilization cavities through an air inflation pipeline, and the air inlet of the air pump is connected with the ports A of the electromagnetic valves and communicates with the outside atmosphere, so that the air inflation cycles are formed.

The air suction pipeline and the air inflation pipeline are each provided with a flow regulating valve, a one-way valve and a sterilizing filter, so that the air cleanliness of the whole pipelines is guaranteed, and secondary pollution of the gloves is prevented.

When the elastic gloves to be donned are fixed to the sleeve fixing rings of the sterilization cavities, the cavity through holes of the sterilization cavities are airtight relative to the outside, and thus a pressure maintaining state is achieved.

The cavity through holes are round or quasi-round or oval or quasi-oval or are of an egg-shaped or quasi-egg-shaped structure with the upper section being sharp and the lower section being obtuse.

The opening edge of each cavity through hole extends towards the two ends in the radial direction from one end of the cavity through hole, and the outer edges of each cavity through hole are in a horn shape or a step shape or a bevel shape; the horn shape refers to that the radius of the opening edge of each cavity through hole becomes large gradually, and the opening edge of each cavity through hole is in smooth arc transition; the step shape refers to that the radius of the opening edge of each cavity through hole becomes larger gradually, and the opening edge of each cavity through hole is in a right-angled step shape; the bevel shape refers to that the radius of the opening edge of the cavity through hole becomes larger gradually, and the opening edge of each cavity through hole is in smooth bevel transition.

The opening edge of each cavity through hole extends towards the two ends in the radial direction from one end of the cavity through hole, and is of an asymmetrical structure with the axis of the cavity through hole as the center.

When the opening edges are of an asymmetrical structure, the opening edges are of the specific structure where the opening edges extend towards the two ends in the radial direction by different dimensions, or the opening edges extend towards the two ends in the radial direction in different shapes, namely one end of the opening edges is of a right-angled step structure, and the other end of the opening edges is of a bevel structure.

The outer edges of the cavity through holes are of a stretchable structure; when the gloves are about to be mounted in the cavity through holes, the cavity through holes contract, and the calibers are decreased; after the gloves are mounted, the cavity through holes expand, the calibers are restored, and the gloves are well fixed. When the gloves are donned on the hands, the calibers of the cavity through holes are decreased again while inflation is conducted for positive pressure, and thus the gloves are released easily.

According to the stretchable structure, the opening edges are made of elastic materials, and elastic pieces are arranged between the opening edges and the cavity through holes; when the gloves are about to be mounted in the cavity through holes, the opening edges move towards the interiors of the cavity through holes under the effect of external force, the elastic pieces are stretched to make the cavity through holes contract, and the calibers are decreased; after the gloves are mounted, the external force disappears, the opening edges restore to the original state under the effect of the elastic pieces, the calibers are restored at the same time, and the gloves are well fixed. When the gloves are donned on the hands, the calibers of the cavity through holes are decreased again while inflation is conducted for positive pressure, and thus the gloves are released easily.

According to the stretchable structure, movable buckle devices are arranged in the positions, close to the opening edges of the outer walls of the cavity through holes, the buckles contract when the gloves are mounted, and oversleeves are fixed through the buckles; before vacuumizing is conducted for negative pressure, the buckles bounce to well fix the gloves; after the gloves are donned, inflation is conducted for positive pressure, the buckles contract again, and the gloves are released easily.

The airtightness of the gloves is detected through positive pressure and negative pressure, namely, vacuumizing is conducted for negative pressure after the gloves are mounted, the gloves are made to expand, the negative pressure of the cavities is detected continuously through pressure sensors in the process, and it indicates that the gloves have holes and cannot be used if the negative pressure of the cavities cannot be achieved or the negative pressure is decreased after the air pump stops. If air leakage is slow and cannot be confirmed, inflation can be conducted for positive pressure, the gloves are made to protrude and expand, the positive pressure is detected through the pressure sensors, and whether the gloves have holes or not can be confirmed through manual observation.

The cavity through holes can be made of different materials and have different dimensions, so that the sleeve fixing rings for the gloves under different stress conditions can be mounted on the cavity through holes in a sleeved mode conveniently and can be dismounted easily under positive pressure after the gloves are donned.

Compared with the prior art, the invention has the following beneficial effects that:

The equipment for elastic glove detecting and donning with the sterilization function of the invention is simple and reasonable in structural design, and the elastic gloves can be stretched to be donned under negative pressure and contracted to be released under positive pressure by controlling the electromagnetic valves through the control cabinet.

The equipment can be used for sterilization of elastic gloves made of various elastic materials, and the elastic gloves can be donned rapidly in an extremely short time; the pressure detection switches are used for detecting the internal pressure of the sterilization cavities, and the air pipelines are used for inflation and deflation so that it can be guaranteed that the gloves are airtight and in the proper state when donned; the wearer controls the switches to generate negative pressure, balanced pressure and positive pressure in the sterilization cavities, so that the elastic gloves are made to contract and automatically fall onto the hands of the wearer from the sleeve fixing rings after being donned; the cleanliness and safety of the equipment and the elastic gloves are guaranteed through physical and chemical sterilization methods including filter sterilization, ozone sterilization and the like, and the sterilization methods are harmless to human bodies. Since the physical and chemical sterilization methods including filter sterilization, ozone sterilization and the like are adopted for the equipment and the air pipelines relevant to glove donning, the sanitation requirement for medical operation can be met.

Furthermore, a pedal switch is used for motion control, so that the wearer is prevented from touching other objects besides the gloves with hands; manual touch control and manual monitoring are not needed during the whole donning process, cross infection can be effectively avoided, and a system is stable and reliable.

Furthermore, the positive pressure threshold value and the negative pressure threshold value of the pressure detection switches can be adjusted to adapt to elastic gloves made of different elastic materials.

Furthermore, in a system control scheme, a two-position switch of the pedal switch and a relay are matched and designed to be of an interlocking form, so that false operation of the system is avoided, and using safety is guaranteed.

### BRIEF DESCRIPTION OF THE SEVERAL VIEWS OF THE DRAWINGS

FIG. 1 is a structural schematic diagram of equipment for elastic glove detecting and donning with a sterilization function of the invention;
FIG. 2 is a structural diagram of the sterilization cavities of the invention;
FIG. 3 is a schematic diagram of the air pipelines in different pressure states of the invention;
FIG. 4 is an outline diagram of cavities through holes;
FIG. 5 is another outline diagram of the cavities through holes;
FIG. 6 is a third outline diagram of the cavities through holes;
FIG. 7 is a structural schematic diagram of the outer edge of the cavity through holes;
FIG. 8 is another structural schematic diagram of the outer edge of the cavity through holes;
FIG. 9 is a third structural schematic diagram of the outer edge of the cavity through holes;
FIG. 10 is a fourth structural schematic diagram of the outer edge of the cavity through holes;
FIG. 11 is a fifth structural schematic diagram of the outer edge of the cavity through hole;
FIG. 12 is a structural schematic diagram of the outer edge, of a stretchable structure, of the cavity through holes, wherein FIG. (a) and FIG. (b) are diagrams in two states correspondingly.
FIG. 13 (a) is a structural schematic diagram of buckles, and FIG. 13 (b) and FIG. 13 (c) are structural schematic diagrams in two states after the buckles are mounted in the cavity through holes correspondingly.

Wherein, 1, sterilization cavity; 2, elastic glove; 3, sleeve fixing ring; 4, air pipeline; 5, air pump; 6, sterilization device; 7, pressure detection switch; 8, electromagnetic valve; 9, flow regulating valve; 10, one-way valve; 11, sterilizing filter; 12, control loop; 13, control cabinet; 14, pedal switch; 15, cavity through hole; 16, cavity shell; 17, air pipeline port.

### DETAILED DESCRIPTION OF THE INVENTION

A detailed description of the invention is given with reference drawings and specific embodiments of the invention as follows.

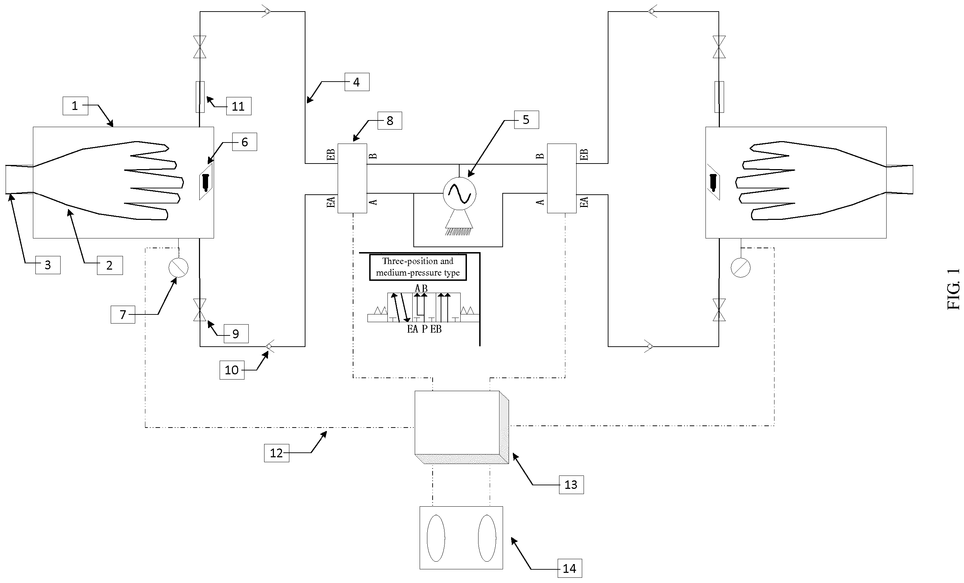

As is shown in FIG. 1, equipment for elastic glove airtightness-detecting and rapid-donning equipment with a sterilization function of the invention comprises two sterilization cavities 1 internally provided with sterilization devices, sleeve fixing rings 3 for elastic gloves 2, connecting air pipelines 4, a two-way air pump 5, ozone generation pieces 6, pressure detection switches 7, electromagnetic valves 8, flow regulating valves 9, one-way valves 10, sterilizing filters 11, control loops 12, a control cabinet 13 and a pedal switch 14. Through holes used for mounting the sleeve fixing rings 3 for the elastic gloves 2 are formed in one side of the sterilization cavities 1; fingerstall sections of the gloves 2 are located inside the sterilization cavities 1, the sleeve ends are fixed to the sterilization cavities 1 through the sleeve fixing rings 3, and the elastic gloves 2 can be donned rapidly under control action; the two sides of the bottom of each sterilization cavity 1 are provided with air pipeline ports 17 which are connected with the two-way air pump 5 serving as an air source, and the small-power ozone generation pieces which serve as the sterilization devices 6 and the pressure detection switches 7 which are used for detecting the internal pressure of the sterilization cavities 1 and used for closed-loop control are also mounted; the small-power ozone generation pieces serve as the internal sterilization devices and are safe, efficient and harmless to human bodies. The air pipelines 4 are provided with the electromagnetic valves 8, the flow regulating valves 9, the one-way valves 10 and the sterilizing filters 11, and are connected to the sterilization cavities 1 and the two-way air pump 5; the air pipelines 4 can be used for controlling the directions and flow rates for air flow in the sterilization cavities 1 and used for sterilizing and filtering.

A module used for action control comprises the control loops 12, the control cabinet 13 and the pedal switch 14. The pedal switch 14 and the pressure detection switches 7 are used for action control and closed-loop control, and manual touch control is not needed.

When the elastic gloves 2 are fixedly mounted on the sleeve fixing rings 3 of the sterilization cavities 1, the cavity through holes are airtight relative to the outside, and elastic deformation of the elastic gloves 2 can be caused by the change of the internal pressure of the cavities. The elastic gloves 2 are made to stretch by means of negative pressure (lower than the standard atmospheric pressure) in the sterilization cavities 1 and thus can be donned easily; when the completeness of the gloves is broken, automatic checking can be conducted through the internal pressure states of the sterilization cavities 1 and the stretching states of the gloves. The elastic gloves 2 are made to contract by means of positive pressure (higher than the standard atmospheric pressure) in the sterilization cavities 1 and thus can be donned rapidly.

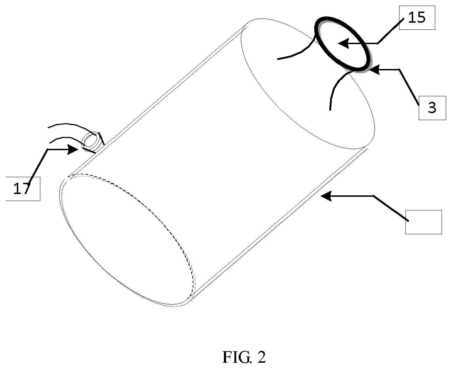

As is shown in FIG. 2, the sterilization cavities used for donning the elastic gloves of the invention structurally comprise the cavity through holes 15, the fixing rings 3, the cavity shells 16 and the air pipeline ports 17. When the elastic gloves to be donned are fixedly mounted on the fixing rings 3, the cavity through holes 15 are airtight relative to the outside; the internal pressure of the cavities can be changed through air flow in the air pipeline ports 17, and elastic deformation of the elastic gloves mounted on the fixing rings 3 is caused accordingly; when the elastic deformation reaches a certain degree, the elastic gloves can be donned conveniently and released automatically after being donned.

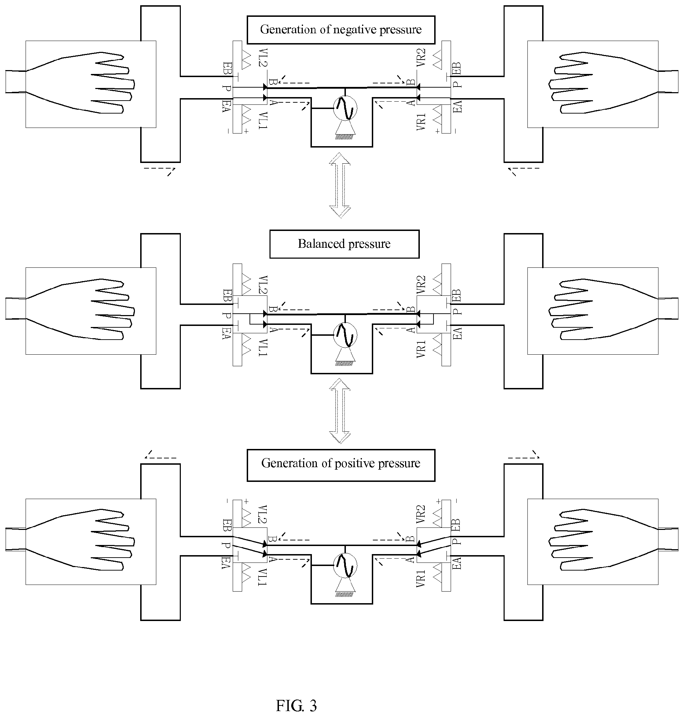

The operating principle of the invention is shown in FIG. 1 and FIG. 3, donning operation is started through the pedal switch 14, and when the internal pressure of the sterilization cavities 1 does not reach the detection threshold value of the pressure detection switches 7, the states of the pipelines are shown by the negative pressure generation part in FIG. 3. At the moment, an air inlet of the two-way air pump 5 is connected with ports A of the two electromagnetic valves 8 and communicates with air outlets of the sterilization cavities 1 through the air pipelines 4, and an air outlet of the two-way air pump 5 is connected with ports B of the two electromagnetic valves 8 and communicates with the outside atmosphere, so that air suction cycles are formed, the pressure in the two sterilization cavities 1 is decreased, and accordingly the elastic gloves 2 are stretched conveniently to be donned. If the elastic gloves 2 are broken at the moment, breakage of the elastic gloves 2 can be checked out automatically through readings on the pressure detection switches 7 and the stretching states of the gloves. When the system is not started through the pedal switch 14, or the pressure detection switches 7 detect that the pressure in the sterilization cavities 1 reaches a certain threshold value, an on-off control signal is output, and the sterilization cavities 1 and the air pipelines 4 are in or switched to a balanced pressure state shown by the balanced pressure part in FIG. 3. The air inlet and the air outlet of the two-way air pump 5 are connected with channels of the electromagnetic valves 8 and both communicate with the outside atmosphere, the air inlets and the air outlets of the sterilization cavities 1 on the left side and the right side are sealed, and the internal air pressure of the cavities can be maintained under a good sealing condition and is equal to the outside air pressure when the cavity through holes are opened. When the wearer treads on the pedal switch 14 on the other side and the internal pressure of the sterilization cavities 1 does not reach the detection threshold value of the pressure detection switches 7, the states of the pipelines is shown by the positive pressure generation part in FIG. 3. The air outlet of the two-way air pump 5 is connected with the ports B of the two electromagnetic valves 8 and communicates with the air inlets of the sterilization cavities 1, and the air inlet of the two-way air pump 5 is connected with the ports A of the electromagnetic valves 8 and communicates with the outside atmosphere, so that air inflation cycles are formed, the pressure in the two cavities is increased, and accordingly the elastic gloves 2 are contracted to be donned.

The small-power ozone generation pieces 6 are used for sterilization in the sterilization cavities 1, safety is guaranteed, and the efficiency is high; the sterilizing filters 11 are mounted on the air pipelines 4 to guarantee the cleanliness of air flow entering and coming out of the cavities, the air flow can be regulated through the flow regulating valves 9 and the one-way valves 10, and working index requirements are met.

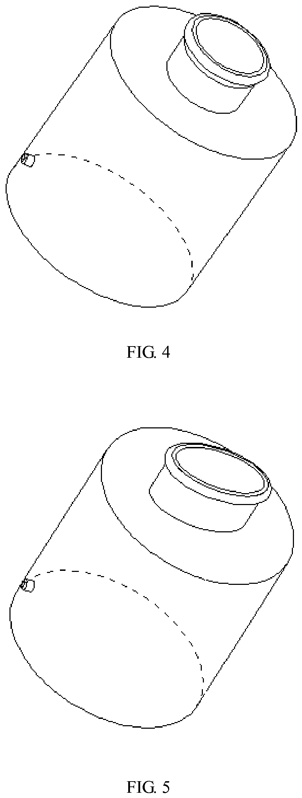

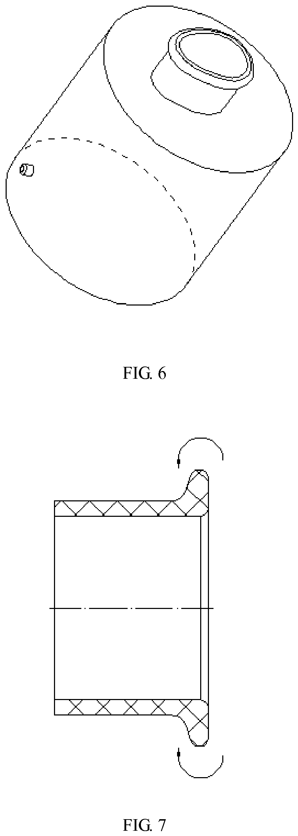

For further guaranteeing that the gloves are donned conveniently through the equipment of the invention, the cross sections of the cavity through holes can be of a round or quasi-round structure (shown in FIG. 4), or of an oval or quasi-oval structure (shown in FIG. 5), or of an egg-shaped or quasi-egg-shaped structure with the upper section being sharp and the lower section being obtuse (shown in FIG. 6).

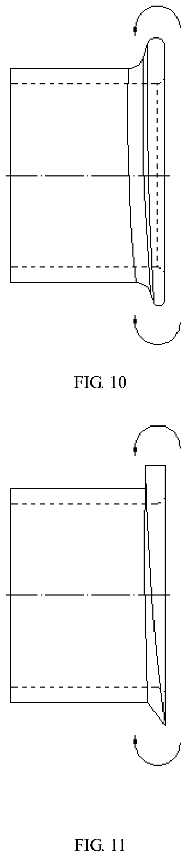

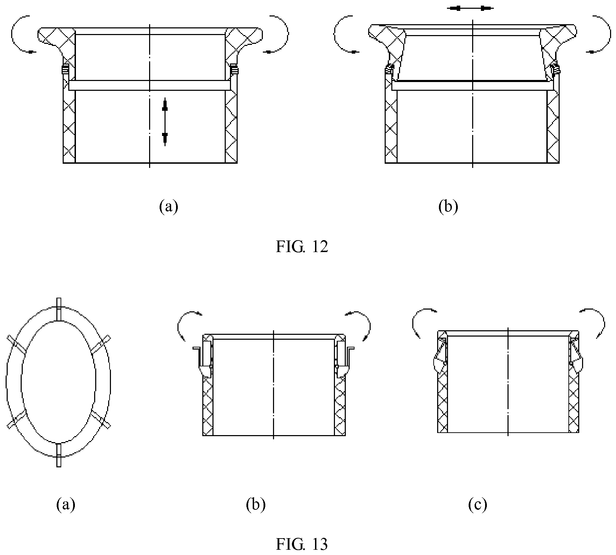

Of course, for donning the gloves conveniently, the following transformation schemes can also be adopted for the cavity through holes:
(1) the scheme where the radius increment of the outer edges is changed (shown in FIG. 10): the radius increment of the opening edges of the cavity through holes is decreased from top to bottom, the radius increment of the upper portions of the opening edges is large so that mounting can be convenient, and the radius increment of the lower portions of the opening edges is small so that dismounting is convenient. In other words, the opening edges of the cavity through holes extend upwards and downwards from one end of the cavity through holes, however, the opening edges extend upwards and downwards by different dimensions, specifically, the upward extension dimension of the opening edges from the center is larger than the downward extension dimension of the opening edges from the center so that after the gloves are placed in the sterilization cavities, the free ends of the gloves can be turned out to be fixedly mounted on the opening edges conveniently, and the gloves can be released easily;
(2) the scheme where the outer edges are changed into a bevel shape from a step shape (shown in FIG. 11): the tops of the opening edges of the cavity through holes are in the step shape, and the opening edges are smoothly changed to be in the bevel shape downwards. The tops of the opening edges are in the right-angled step shape so that mounting can be convenient, and the bottoms of the opening edges are in the bevel shape so that dismounting can be easy. In other words, the opening edges of the cavity through holes extend upwards and downwards from one end of the cavity through holes, however, the upward extension parts of the opening edges are designed to be right-angled, and the downward extension parts of the opening edges are designed to be bevel;
(3) the scheme where the outer edges are of a stretchable mode (shown in FIG. 12): movable cavity through holes are adopted; in one embodiment of the invention, the opening edges are made of elastic materials, and elastic pieces are arranged between the opening edges and the cavity through holes; when the gloves are about to be mounted in the cavity through holes, the opening edges move towards the interiors of the cavity through holes under the effect of external force, the elastic pieces are stretched at the same time, the cavity through holes contract, the calibers are decreased, and the gloves can be mounted conveniently; after the gloves are mounted, the external force disappears, the opening edges restore to the original state under the effect of the elastic pieces, the calibers are restored at the same time, and the gloves are well fixed. The gloves are donned after being expanded under negative pressure, then balanced pressure and positive pressure are achieved, and after the positive pressure reaches a certain pressure value, the cavity through holes contract again for releasing the gloves easily.

For mounting the gloves more conveniently and releasing the gloves more easily, movable buckle devices (shown in FIG. 13) are arranged in the positions, close to the opening edges, of the outer walls of the cavity through holes; when the gloves are mounted, buckles contract, and oversleeves are fixed through the buckles; before vacuumizing for negative pressure, the buckles bounce to well fix the gloves; after the gloves are donned, inflation is conducted for positive pressure, the buckles contract again, and the gloves.

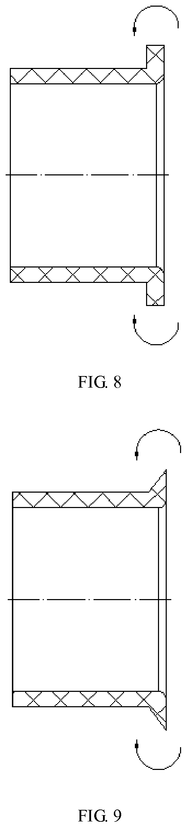

The outer edges of the cavity through holes can also be of a horn-shaped structure or a step-shaped structure or a bevel-shaped structure; as is shown in FIG. 7, the horn shape refers to that the radiuses of the opening edges of the cavity through holes are increased gradually, and the opening edges are in smooth bevel transition; as is shown in FIG. 8, the step shape refers to that the radiuses of the cavity through holes are increased, and the opening edges are in a right-angled step shape; as is shown in FIG. 9, the bevel shape refers to that the radiuses of the cavity through holes are increased gradually, the opening edges are in smooth bevel transition.

The equipment for elastic glove airtightness-detecting and rapid-donning with the sterilization function of the invention is simple and reasonable in structural design, the principle basis is sufficient, the technical scheme can be achieved easily, the equipment is safe, convenient to use and efficient, and the requirement for the sanitation condition in elastic glove donning operation can be fully met.

## Claims

1. Equipment for elastic glove detecting and donning with a sterilization function, **characterized by** comprising sterilization cavities (1), wherein elastic gloves (2) are fixedly mounted in the sterilization cavities, fingerstall sections of the elastic gloves (2) are located inside the sterilization cavities (1), cavity through holes (15) used for mounting sleeve fixing rings (3) are formed in one side of the sterilization cavities (1), and the sleeve ends are fixed to the sterilization cavities (1) through the sleeve fixing rings (3); the sterilization cavities (1) are further provided with pressure detection switches (7) which are used for detecting the internal pressure of the sterilization cavities (1) and conducting closed-loop control; the sterilization cavities (1) are further provided with air pipeline ports (17) used for being connected with an air pump (5), the air pump (5) is connected with the sterilization cavities (1) through electromagnetic valves (8), the sterilization cavities (1) and the electromagnetic valves (8) are further connected with a control cabinet (13), and the electromagnetic valves (8) are controlled through the control cabinet (13), so that the elastic gloves (2) are stretched to be donned under negative pressure and contracted to be released under positive pressure.

2. The equipment for elastic glove airtightness-detecting and rapid-donning with the sterilization function according to Claim 1, **characterized in that** the elastic glove airtightness-detecting and rapid-donning equipment with the sterilization function further comprises air suction cycles used for forming negative-pressure areas in the airtight areas between the interiors of the sterilization cavities and the exteriors of the gloves to make the gloves expand to become larger, and air inflation cycles used for forming positive-pressure areas in the airtight areas between the interiors of the sterilization cavities and the exteriors of the gloves to attach the gloves completely to human hands; an air inlet of the air pump (5) is connected with ports A of the electromagnetic valves (8) and communicates with air outlets of the sterilization cavities (1) through an air suction pipeline, and an air outlet of the air pump (5) is connected with ports B of the electromagnetic valves (8) and communicates with the outside atmosphere, so that the air suction cycles are formed; the air outlet of the air pump (5) is connected with the ports B of the electromagnetic valves (8) and communicates with air inlets of the sterilization cavities (1) through an air inflation pipeline, and the air inlet of the air pump (5) is connected with the ports A of the electromagnetic valves (8) and communicates with the outside atmosphere, so that the air inflation cycles are formed.

3. The equipment for elastic glove detecting and donning with the sterilization function according to Claim 2, **characterized in that** the air suction pipeline and the air inflation pipeline are each provided with a flow regulating valve (9), a one-way valve (10) and a sterilizing filter (11), so that the air cleanliness of the whole pipelines is guaranteed, and secondary pollution of the gloves is prevented.

4. The equipment for elastic glove detecting and donning with the sterilization function according to Claim 1, **characterized in that** when the elastic gloves to be donned are fixed to the sleeve fixing rings (3) of the sterilization cavities (1), the cavity through holes (15) of the sterilization cavities (1) are airtight relative to the outside, and thus a pressure maintaining state is achieved.

5. The equipment for elastic glove detecting and donning with the sterilization function according to Claim 1, **characterized in that** the opening edge of each cavity through hole (15) extends towards the two ends in the radial direction from one end of the cavity through hole, and the outer edge of each cavity through hole (15) is in a horn shape or a step shape or a bevel shape; the horn shape refers to that the radius of the opening edge of each cavity through hole becomes large gradually, and the opening edge of each cavity through hole is in smooth arc transition; the step shape refers to that the radius of the opening edge of each cavity through hole becomes larger gradually, and the opening edge of each cavity through hole is in a right-angled step shape; the bevel shape refers to that the radius of the opening edge of the cavity through hole becomes larger gradually, and the opening edge of each cavity through hole is in smooth bevel transition.

6. The equipment for elastic glove detecting and donning with the sterilization function according to Claim 1, **characterized in that** the opening edge of each cavity through hole (15) extends towards the two ends in the radial direction from one end of the cavity through hole, and is of an asymmetrical structure with the axis of the cavity through hole as the center.

7. The equipment for elastic glove detecting and donning with the sterilization function according to Claim 6, **characterized in that** when the opening edges are of an asymmetrical structure, the opening edges are of the specific structure where the opening edges extend towards the two ends in the radial direction by different dimensions, or the opening edges extend towards the two ends in the radial direction in different shapes, namely one end of the opening edges is of a right-angled step structure, and the other end of the opening edges is of a bevel structure.

8. The equipment for elastic glove detecting and donning with the sterilization function according to Claim 1, **characterized in that** the outer edges of the cavity through holes are of a stretchable structure; when the gloves are about to be mounted in the cavity through holes, the cavity through holes contract, and the calibers are decreased; after the gloves are mounted, the cavity through holes expand, the calibers are restored, and the gloves are well fixed.

9. The equipment for elastic glove detecting and donning with the sterilization function according to Claim 8, **characterized in that** according to the stretchable structure, the opening edges are made of elastic materials, and elastic pieces are arranged between the opening edges and the cavity through holes; when the gloves are about to be mounted in the cavity through holes, the opening edges move towards the interiors of the cavity through holes under the effect of external force, the elastic pieces are stretched to make the cavity through holes contract, and the calibers are decreased; after the gloves are mounted, the external force disappears, the opening edges restore to the original state under the effect of the elastic pieces, the calibers are restored at the same time, and the gloves are well fixed.

10. The equipment for elastic glove detecting and donning with the sterilization function according to Claim 8, **characterized in that** according to the stretchable structure, movable buckle devices are arranged in the positions, close to the opening edges, of the outer walls of the cavity through holes, the buckles contract when the gloves are mounted, and sleeves are fixed through the buckles; before vacuumizing is conducted for negative pressure, the buckles bounce to well fix the gloves; after the gloves are donned, inflation is conducted for positive pressure, the buckles contract again, and the gloves are released easily.
